# EUROPÄISCHE PATENTANMELDUNG

(11) **EP 0 739 654 A2**
(43) Veröffentlichungstag der Anmeldung: **30.10.1996**
(21) Anmeldenummer: 96112316.3
(22) Anmeldetag: 13.05.1992
(51) Int. Cl.: B05B 17/06

(54) **Zerstäubungsgerät, insbesondere Taschen-Zerstäubungsgerät**

(62) Teilanmeldung aus: 92108101.4
(71) Anmelder: SIEMENS AKTIENGESELLSCHAFT, 80333 München (DE)
(72) Erfinder: van der Linden, Klaus, 96450 Coburg (DE)

(57) **Zusammenfassung**

Das Zerstäubungsgerät (1) hat ein Gehäuse (2), einen Aufsatz (4) zum Gehäuse (2) mit einem Vorratsbehälter (30, 64) für eine zu zerstäubende Flüssigkeit (F), einen Ultraschallzerstäuber (6), eine elektrische Stromversorgungseinrichtung (14), eine Einrichtung zur Beförderung der zu zerstäubenden Flüssigkeit (F) zum Ultraschallzerstäuber (6) sowie ein Auslöseelement (10). Dabei umfaßt die Einrichtung zur Beförderung der Flüssigkeit (F) eine Antriebsquelle, mit der unabhängig von der Einwirkungskraft und -dauer auf das Auslöseelement (10) eine vorgebbare Menge Flüssigkeit (F) beförderbar ist, sowie einen Regler (16, 60), der die zu dosierende Menge Flüssigkeit (F) überwacht und dem eine mit dem Vorratsbehälter (30,64) verknüpfte Codiereinrichtung (34,36) zugeordnet ist.

## Beschreibung

Die Erfindung bezieht sich auf ein Zerstäubungsgerät, insbesondere ein Taschen-Zerstäubungsgerät zur Zerstäubung von Arzneimitteln für Inhalationszwecke, mit einem Gehäuse, einem Aufsatz zum Gehäuse mit einem Vorratsbehälter für eine zu zerstäubende Flüssigkeit, einem Ultraschallzerstäuber, einer elektrischen Stromversorgungseinrichtung sowie einer Einrichtung zur Beförderung der zu zerstäubenden Flüssigkeit und einem Auslöseelement.

Ein solches Zerstäubungsgerät geht hervor aus der EP 0 111 163 A2.

In vielen Fällen ist es wünschenswert, eine Flüssigkeit zu zerstäuben. Beispiele hierfür sind im kosmetischen Bereich die Zerstäubung eines Haarlackes und im medizinischen Bereich die Zerstäubung eines Arznei- oder Desinfektionsmittels. Handelsübliche Zerstäubungsgeräte, insbesondere zur Inhalation von Arzneimitteln, wie Sprüh- und Spritzpistolen und mechanische Handvernebler, haben den Nachteil, daß mit diesen keine hinreichend feine Verteilung und Homogenität des Aerosols erreichbar ist. Zudem ist bei den mechanischen Handverneblern ein großer Kraftaufwand erforderlich. Alternativ als Zerstäubungsgeräte einsetzbare Aerosol-Sprühdosen haben insbesondere bei der Anwendung zur Inhalation eines Arzneimittels den Nachteil, daß bei der Applikation ein Kältereiz auftreten kann und die Treibgase eine zum Teil schädliche Wirkung haben. Des weiteren führen die sehr hohen Geschwindigkeiten des Aerosols zu Problemen bei der Koordination der Betätigung der Sprühdose und dem Inhalieren durch den Patienten.

Ein Zerstäubungsgerät mit einer von Hand zu bedienenden Schlauchpumpe zur exakten Dosierung eines Inhalats geht aus der DE 35 08 560 A1 hervor.

Für die Zerstäubung von Flüssigkeiten sind auch Ultraschallzerstäuber geeignet. Solche Ultraschallzerstäuber, die beispielsweise als Tascheninhalationsgeräte ausgeführt sind, sind aus der EP 0 258 637 B1 und aus der EP 0 373 237 A1 bekannt.

Diese Zerstäubungsgeräte umfassen ein Gehäuse, in dem unter anderem ein Ultraschallzerstäuber und eine elektrische Stromversorgungseinrichtung angeordnet sind, sowie einen Aufsatz zum Gehäuse, in dem ein Vorratsbehälter für eine zu zerstäubende Flüssigkeit angeordnet ist. Zur Betätigung dieser Ultraschallzerstäuber ist eine Krafteinwirkung des Bedieners auf ein Auslöseelement erforderlich, wodurch eine durch die Dimensionierung einer Dosierkammer vorgegebene Flüssigkeitsmenge zu einem Zerstäuberteller geführt wird. Auf dem Zerstäuberteller wird diese Flüssigkeitsmenge zu einem, z.B. lungengängigen, Aerosol zerstäubt. Bekannte Dosiersysteme, die sich als Einrichtung zur Beförderung der Flüssigkeit z.B. eines Schleppkolbens (EP 0 258 637 B1) oder einer Drucktaste (FR 2 444 504 A1) bedienen, haben den Nachteil, daß Fehldosierungen aufgrund der unterschiedlichen Krafteinwirkung des Bedieners auf das Auslöseelement auftreten. Zum anderen kann die Dosiermenge nicht an unterschiedliche zu zerstäubende Flüssigkeiten und deren Medikation angepaßt werden.

Der Erfindung liegt daher die Aufgabe zugrunde, ein Zerstäubungsgerät, insbesondere ein Taschen-Zerstäubungsgerät, anzugeben, mit dem es möglich ist, Fehldosierungen zu vermeiden und verschiedene vorgebbare Flüssigkeitsmengen zu dosieren.

Diese Aufgabe wird erfindungsgemäß gelöst durch ein Zerstäubungsgerät, insbesondere Taschen-Zerstäubungsgerät, mit:
a) einem Gehäuse;
b) einem Aufsatz zum Gehäuse, mit einem Vorratsbehälter für eine zu zerstäubende Flüssigkeit;
c) einem Ultraschallzerstäuber;
d) einer elektrischen Stromversorgungseinrichtung;
e) einer Einrichtung zur Beförderung der zu zerstäubenden Flüssigkeit zum Ultraschallzerstäuber; und
f) einem Auslöseelement; bei dem
g) die Einrichtung zur Beförderung der Flüssigkeit eine Antriebsquelle umfaßt, mit der unabhängig von der Einwirkungskraft und -dauer auf das Auslöseelement eine vorgebbare Menge Flüssigkeit beförderbar ist; und
h) die Einrichtung zur Beförderung der Flüssigkeit einen Regler umfaßt, der die zu dosierende Menge Flüssigkeit überwacht und dem eine mit dem Vorratsbehälter verknüpfte Codiereinrichtung zugeordnet ist.

Bei diesem Zerstäubungsgerät werden Fehldosierungen vermieden, da ein Bediener des Zerstäubungsgeräts keine Kraft zur Beförderung der Flüssigkeit aufbringen muß.

Mittels der Codiereinrichtung wird dem Regler des Zerstäubungsgeräts ein Sollwert vorgegeben, der der zu dosierenden Flüssigkeitsmenge entspricht und auf die im Vorratsbehälter befindliche Flüssigkeit abgestimmt ist.

Es ist besonders zweckmäßig, daß der Regler die Einrichtung zur Beförderung der Flüssigkeit abschaltet, sobald die geförderte Flüssigkeitsmenge den Wert erreicht, den die mit dem Vorratsbehälter verknüpfte Codiereinrichtung vorgibt. Dadurch ist eine genaue Dosierung einer vorgegebenen Flüssigkeitsmenge gewährleistet, denn die Angleichung des Ist-Wertes der mittels der Antriebsquelle geförderten Flüssigkeit an den vorgegebenen Soll-Wert hängt nicht von der Krafteinwirkung des Bedieners ab, sondern wird von dem Regler gesteuert.

In besonders vorteilhafter Weiterbildung der Erfindung kann die Antriebsquelle die Flüssigkeit mittels Druckeinwirkung befördern. Damit ist das Zerstäubungsgerät lageunabhängig betreibbar.

Zur Entkopplung der Antriebsquelle von einer elektrischen Stromversorgungseinrichtung kann die Flüssigkeit vorteilhafterweise über eine elastische Kraft unter Druck gesetzt werden.

Zweckmäßigerweise kann die Flüssigkeit über eine auf einen Kolben wirkende Federkraft unter Druck gesetzt werden.

Eine besonders zweckmäßige Weiterbildung der Erfindung sieht eine Pumpe als Einrichtung zur Beförderung der Flüssigkeit vor. Eine Pumpe eignet sich besonders für die Förderung verschiedener, vorgebbarer Flüssigkeitsmengen, da die Pumpe bezüglich ihrer Drehzahl und der Gesamtzahl der Umdrehungen einfach regelbar ist.

In Weiterbildung der Erfindung kann die Einrichtung zur Beförderung der Flüssigkeit diese bei abgeschaltetem Gerät gegenüber der Umgebungsluft abdichten. Hierdurch wird ein Austrocknen und/oder Auslaufen der Flüssigkeit bei längerem Nichtgebrauch vermieden und die chemische Reaktion von Umgebungsluft und Arneimittel unterbunden.

Als Einrichtung zur Beförderung der Flüssigkeit kann vorteilhafterweise eine Schlauchpumpe verwendet sein. Weil so die zu befördernde Flüssigkeit nur mit dem die Flüssigkeit führenden Schlauch in Berührung kommt, eignet sich eine Schlauchpumpe besonders zur sterilen Beförderung einer Flüssigkeit, insbesondere eines Arzneimittels.

Ein vorteilhafter Aufbau des Zerstäubungsgeräts wird erreicht, wenn in dem Gehäuse der Ultraschallzerstäuber, die elektrische Stromversorgungseinrichtung und das Auslöseelement eingebaut sind, im Aufsatz zum Gehäuse der Vorratsbehälter und eine mit dem Vorratsbehälter verknüpfte Codiereinrichtung eingebaut sind und die Einrichtung zur Beförderung der zu zerstäubenden Flüssigkeit mit Ausnahme eines im Aufsatz befindlichen, den luftdichten Abschluß der Flüssigkeit bewirkenden Elementes im Gehäuse eingebaut ist. Hierdurch ist es dem Bediener möglich, das Zerstäubungsgerät mit verschiedenen auswechselbaren Aufsätzen zum Gehäuse zu betreiben. Dabei ist die Flüssigkeit in jedem Aufsatz zum Gehäuse gegenüber der Umgebungsluft abgedichtet und für jeden Aufsatz zum Gehäuse eine variable, zu dosierende Flüssigkeitsmenge mittels der zugehörigen Codiereinrichtung vorgegeben.

Ausführungsbeispiele der Erfindung werden anhand der Zeichnung näher erläutert. Dabei zeigen:
- Figur 1: einen Längsschnitt durch ein erfindungsgemäßes Zerstäubungsgerät, hier ein Inhalationsgerät für Arzneimittel; und
- Figur 2: einen vergrößerten Ausschnitt aus einem anderen erfindungsgemäßen Zerstäubungsgerät.

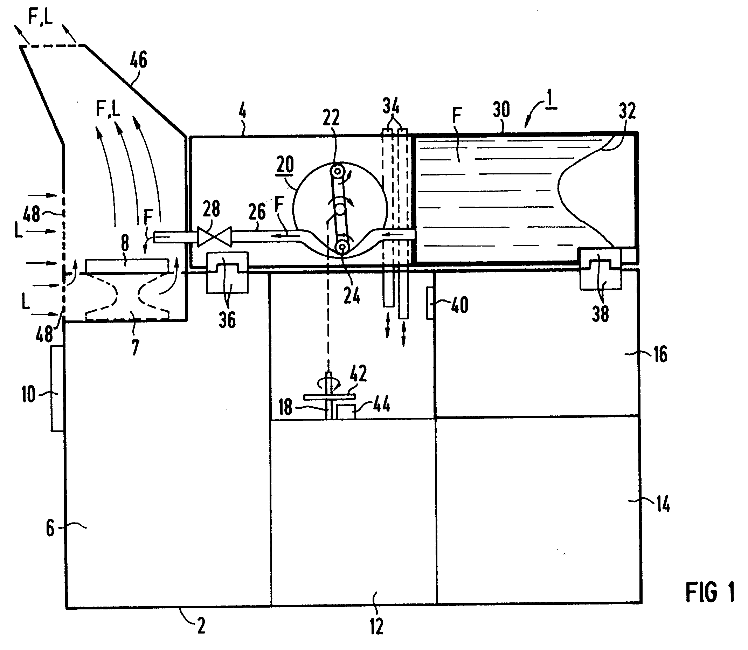

Das in Figur 1 dargestellte Inhalationsgerät 1 umfaßt ein Gehäuse 2 und einen Aufsatz 4 zum Gehäuse 2. Das Gehäuse 2 enthält unter anderem einen Ultraschallzerstäuber 6 mit einem Amplitudentransformator 7, dessen eine Oberfläche als Zerstäuberteller 8 ausgebildet ist, ein Auslöseelement 10, einen Motor 12, eine elektrische Versorgungseinrichtung 14, einen Regler 16 und eine Motorwelle 18. In dem Aufsatz 4 zum Gehäuse 2 ist ein Vorratsbehälter 30 mit der zu zerstäubenden Flüssigkeit F und einem Faltsack 32, eine Codiereinrichtung in Form von Codierstiften 34, eine Schlauchpumpe 20 mit ihren Druckrollen 22 und 24, ein Schlauch 26 sowie ein Schlauchventil 28 eingebaut. Der Aufsatz 4 ist mittels der Magnethalterungen 36 und 38 am Gehäuse 2 auswechselbar befestigt. Außerdem ist im Gehäuse 2 ein Sensor 40 zur Abtastung der Position der Codierstifte 34 vorgesehen. Auf die Motorwelle 18 ist eine Segmentscheibe 42 montiert, deren Drehwinkel mit einem weiteren Sensor 44 abgetastet wird. Bei auf das Gehäuse 2 aufgesetztem Aufsatz 4 kuppelt, wie dargestellt, die Motorwelle 18 an die Schlauchpumpe 20 an, der Schlauch 26 ragt in den Raum unmittelbar über dem Zerstäuberteller 8 und die Codierstifte 34 ragen in das Gehäuse 2. Zur Inhalation der zu zerstäubenden Flüssigkeit F sind ein Mundstück 46 und Lufteinlaßöffnungen 48 im Gehäuse 2 vorgesehen.

Nach der Betätigung des Auslöseelements 10 - im vorliegenden Fall ein kapazitiver Schalter - wertet der Regler 16 mittels des Sensors 40 die Position der Codierstifte 34, mit denen die zu dosierende Flüssigkeitsmenge vorgebbar ist, aus. Unabhängig von der Einwirkungskraft und -dauer auf das Auslöseelement 10 startet der Regler 16 den Motor 12 und den Ultraschallzerstäuber 6 mit seinem Zerstäuberteller 8 und öffnet das Schlauchventil 28. Der Motor 12 treibt über die Motorwelle 18 die Schlauchpumpe 20 an, die mittels der rotierenden Druckrollen 22 und 24 die Flüssigkeit F aus dem Vorratsbehälter 30 zum Zerstäuberteller 8 fördert. Die zu dosierende Flüssigkeitsmenge, die mit den Codierstiften 34 vorgegeben ist, wird mittels der auf der Motorwelle 18 montierten Segmentscheibe 42 und des Sensors 44 von dem Regler 16 überwacht. Die zu dosierende Flüssigkeitsmenge hängt hierbei von der Anzahl der Umdrehungen der Schlauchpumpe 20, dem Durchmesser des Schlauchs 26 und der Elastizität des Schlauchs 26 ab.

Stellt der Regler 16 die Übereinstimmung der von den Codierstiften 34 vorgegebenen Flüssigkeitsmenge mit der von der Schlauchpumpe 20 geförderten Flüssigkeitsmenge fest, schaltet der Regler den Motor 12 ab und schließt das Schlauchventil 28. Die von der Schlauchpumpe 20 geförderte Flüssigkeitsmenge wird auf dem Zerstäuberteller 8 zerstäubt. Saugt der Patient über das Mundstück 46 Luft L durch die Lufteinlaßöffnungen 48 an, wird das sich über dem Zerstäuberteller 8 bildende Aerosol mit der angesaugten Luft L inhaliert. Die mittels der Codierstifte 34 vorgegebene Flüssigkeitsmenge ist an die jeweils im Vorratsbehälter 30 befindliche Flüssigkeit F und deren Dosiermenge angepaßt. Abweichend von dem in Figur 1 dargestellten Ausführungsbeispiel kann die Schlauchpumpe 20 auch so in den Aufsatz 4 zum Gehäuse 2 eingebaut werden, daß ein Normalenvektor einer Ebene, die durch die Drehbewegung der Schlauchpumpe 20 aufgespannt wird, parallel zur Motorwelle 18 orientiert ist. Auf diese Weise kann auf das in Figur 1 nicht weiter dargestellte Getriebe, das in Figur 1 notwendigerweise zwischen die Motorwelle 18 und die Schlauchpumpe 20 geschaltet werden muß, verzichtet werden.

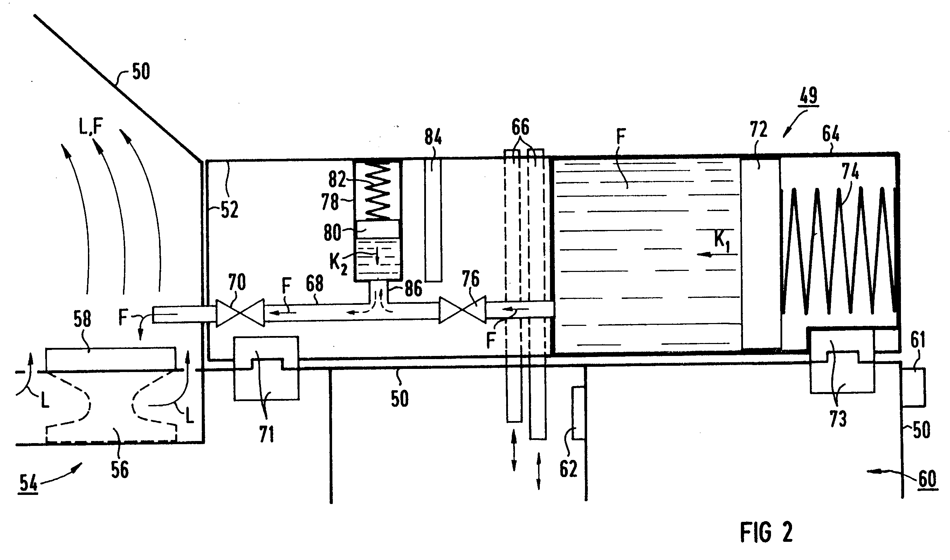

Der in Figur 2 dargestellte Ausschnitt aus einem anderen Zerstäubungsgerät 49 zeigt eine alternative Art zur Beförderung der Flüssigkeit F. Das in der Figur 2 im Ausschnitt dargestellte Zerstäubungsgerät umfaßt - ähnlich wie dies anhand der Figur 1 gezeigt wurde - ein Gehäuse 50 und einen Aufsatz 52 zum Gehäuse 50. Das Gehäuse 50 enthält unter anderem einen Ultraschallzerstäuber 54 mit einem Amplitudentransformator 56, dessen eine Oberfläche als Zerstäuberteller 58 ausgebildet ist, einen Regler 60 und einen Sensor 62. In den Aufsatz 52 zum Gehäuse 50 ist unter anderem ein Vorratsbehälter 64 mit der zu zerstäubenden Flüssigkeit, eine Codiereinrichtung in Form von Codierstiften 66, sowie ein vom Vorratsbehälter 64 zum Zerstäuberteller 58 führender Schlauch 68 mit einem Schlauchventil 70 eingebaut. Abweichend von Figur 1 ist jedoch im Aufsatz 52 ein Kolben 72 im Vorratsbehälter 64, eine mit der Federkraft K₁ auf den Kolben 72 wirkende Feder 74, ein weiteres Schlauchventil 76, ein Zwischenbehälter 78 mit einem Kolben 80 und einer mit der Federkraft K₂ auf den Kolben 80 wirkenden Feder 82 im Zwischenbehälter 78 eingebaut. Der Zwischenbehälter 78 ist mittels eines T-Stücks 86 zwischen den Schlauchventilen 70 und 76 an den zum Zerstäuberteller 58 führenden Schlauch 68 angeschlossen.

Beim Betrieb des Zerstäubungsgerätes 49 öffnet der Regler 60 nach Betätigung eines Auslöseelements das ausgangsseitig am Vorratsbehälter 64 angeordnete Schlauchventil 76 bei gleichzeitig geschlossenem Schlauchventil 70. Mittels der Druckeinwirkung der Feder 74, die mit der Kraft K₁ auf den Kolben 72 des Vorratsbehälters 64 wirkt, wird die zu zerstäubende Flüssigkeit F gegen die auf den Kolben 80 wirkende Federkraft K₂ in Zwischenbehälter 78 gedrückt. Die Position des Kolbens 80 in dem Zwischenbehälter 78 wird hierbei von dem Sensor 84 abgetastet und von dem Regler 60 erfaßt. Erreicht die Position des Kolbens 80 in dem Zwischenbehälter 78 ausgehend von der Ausgangsposition die Position, die der mittels der Codierstifte 66 vorgegebenen Flüssigkeitsmenge entspricht, schließt der Regler 60 das am Ausgang des Vorratsbehälters 64 angeordnete Schlauchventil 76. Gleichzeitig öffnet er das dem Zerstäuberteller 58 unmittelbar vorgeschaltete Schlauchventil 70. Die zu dosierende Flüssigkeitsmenge wird mittels der Druckeinwirkung der Feder 82, die mit der Kraft K₂ auf den Kolben 80 des Zwischenbehälters 78 wirkt, zum Zerstäuberteller 58 des Ultraschallzerstäubers 54 gefördert. Nachdem die zu dosierende Flüssigkeitsmenge aus dem Zwischenbehälter 78 befördert ist, schließt der Regler 60 das Ventil 70. Auf diese Weise ist, ebenso wie in dem in Figur 1 gezeigten Fall, unabhängig von der Auslösekraft und -dauer eine vorgebbare Flüssigkeitsmenge beförderbar.

Es wäre auch denkbar, den Sensor 84 am Zwischenbehälter 78 wegzulassen und den Kolben 80 bei jedem Förderhub zwischen zwei Anschlägen im Zwischenbehälter 78 zu verstellen. In diesem Fall wäre nur das Volumen zwischen den beiden Anschlägen dosierbar. Diese Konstruktion zeichnet sich durch einen Preisvorteil aus.

Des weiteren wäre es möglich, ganz auf den Zwischenbehälter 78 und das Schlauchventil 76 zu verzichten und über einen anderen Sensor die Verstellung des Kolbens 72 im Vorratsbehälter 64 abzutasten, um mit den Signalen den Regler 60 anzusteuern und über den Regler 60 das Schlauchventil 76 zu steuern.

## Patentansprüche

1. Zerstäubungsgerät, insbesondere Taschen-Zerstäubungsgerät, mit:
a) einem Gehäuse (2);
b) einem Aufsatz (4) zum Gehäuse (2), mit einem Vorratsbehälter (30,64) für eine zu zerstäubende Flüssigkeit (F);
c) einem Ultraschallzerstäuber (6);
d) einer elektrischen Stromversorgungseinrichtung (14);
e) einer Einrichtung zur Beförderung der zu zerstäubenden Flüssigkeit (F) zum Ultraschallzerstäuber (6); und
f) einem Auslöseelement (10);
**dadurch gekennzeichnet,** daß
g) die Einrichtung zur Beförderung der Flüssigkeit (F) eine Antriebsquelle umfaßt, mit der unabhängig von der Einwirkungskraft und -dauer auf das Auslöseelement (10) eine vorgebbare Menge Flüssigkeit (F) beförderbar ist; und
h) die Einrichtung zur Beförderung der Flüssigkeit (F) einen Regler (16,60) umfaßt, der die zu dosierende Menge Flüssigkeit (F) überwacht und dem eine mit dem Vorratsbehälter (30,64) verknüpfte Codiereinrichtung (34,66) zugeordnet ist.

2. Zerstäubungsgerät nach Anspruch 1, **dadurch gekennzeichnet,** daß die Antriebsquelle die Flüssigkeit (F) mittels Druckeinwirkung befördert.

3. Zerstäubungsgerät nach Anspruch 1 oder 2, **dadurch gekennzeichnet,** daß die Flüssigkeit (F) über eine elastische Kraft unter Druck setzbar ist.

4. Zerstäubungsgerät nach einem der Ansprüche 1 bis 3, **dadurch gekennzeichnet,** daß die Flüssigkeit (F) über eine auf einen Kolben (72) wirkende Federkraft (K₁) unter Druck setzbar ist.

5. Zerstäubungsgerät nach Anspruch 1, **dadurch gekennzeichnet,** daß als Antriebsquelle für die Beförderung der Flüssigkeit (F) die Schwerkraft dient.

6. Zerstäubungsgerät nach Anspruch 1 oder 2, **dadurch gekennzeichnet,** daß die Einrichtung zur Beförderung der Flüssigkeit (F) eine Pumpe ist.

7. Zerstäubungsgerät nach einem der Ansprüche 1, 2 oder 6, **dadurch gekennzeichnet,** daß die Einrichtung zur Beförderung der Flüssigkeit (F) diese bei abgeschaltetem Gerät (1) gegenüber der Umgebungsluft (L) abdichtet.

8. Zerstäubungsgerät nach einem der Ansprüche 1, 2, 3, 6 und 7, **dadurch gekennzeichnet,** daß die Einrichtung zur Beförderung der Flüssigkeit (F) eine Schlauchpumpe (20) ist.

9. Zerstäubungsgerät nach einem der Ansprüche 1 bis 8, **dadurch gekennzeichnet,** daß dem Regler (16,60) eine mit der Einrichtung zur Beförderung der Flüssigkeit (F) verknüpfte Codiereinrichtung (42,84) zugeordnet ist.

10. Zerstäubungsgerät nach einem der vorhergehenden Ansprüche, **dadurch gekennzeichnet,** daß die Codiereinrichtung (34,66) eine mechanisch abtastbare Struktur ist.

11. Zerstäubungsgerät nach einem der Ansprüche 1 bis 9, **dadurch gekennzeichnet,** daß die Codiereinrichtung (34,66) optisch abtastbar ist.

12. Zerstäubungsgerät nach einem der Ansprüche 1 bis 9, **dadurch gekennzeichnet,** daß die Codiereinrichtung (34,66) elektromagnetisch abtastbar ist.

13. Zerstäubungsgerät nach einem der Ansprüche 1 bis 12, **dadurch gekennzeichnet,** daß der Regler (16,60) die Einrichtung zur Beförderung der Flüssigkeit (F) abschaltet, sobald die geförderte Flüssigkeitsmenge den Wert erreicht, den die mit dem Vorratsbehälter (30,64) verknüpfte Codiereinrichtung (34,66) vorgibt.

14. Zerstäubungsgerät nach einem der Ansprüche 1 bis 13, **dadurch gekennzeichnet,** daß in dem Gehäuse (2) der Ultraschallzerstäuber (6), die elektrische Stromversorgungseinrichtung (14) und das Auslöseelement (10) eingebaut sind, im Aufsatz (4) zum Gehäuse (2) der Vorratsbehälter (30) und eine mit dem Vorratsbehälter (30) verknüpfte Codiereinrichtung (34) eingebaut sind und die Einrichtung zur Beförderung der zu zerstäubenden Flüssigkeit (F) mit Ausnahme eines im Aufsatz (4) befindlichen, den luftdichten Abschluß der Flüssigkeit bewirkenden Elements (22,24) im Gehäuse (2) eingebaut ist.
